# EUROPÄISCHE PATENTSCHRIFT

(11) **EP 3 119 459 B1**
(45) Veröffentlichungstag und Bekanntmachung des Hinweises auf die Patenterteilung: **24.01.2018**
(21) Anmeldenummer: 15703550.2
(22) Anmeldetag: 30.01.2015
(51) Int. Cl.: B05B 12/00, B05B 11/00, A61M 15/00, G06F 19/00

(54) **SPENDER MIT EINER ELEKTRONISCHEN BETÄTIGUNGSERKENNUNG**
DISPENSER HAVING ELECTRONIC ACTUATION DETECTION
DISTRIBUTEUR À RECONNAISSANCE ÉLECTRONIQUE D'ACTIONNEMENT

(30) Priorität: 17.03.2014 DE 102014204939
(43) Veröffentlichungstag der Anmeldung: 25.01.2017
(73) Patentinhaber: Aptar Radolfzell GmbH, 78315 Radolfzell (DE)
(72) Erfinder: KÖRNER, Joachim, 88690 Uhldingen-Mühlhofen (DE)
(74) Vertreter: Patentanwaltskanzlei Cartagena
(86) Internationale Anmeldenummer: PCT/EP2015/051985
(87) Internationale Veröffentlichungsnummer: WO 2015/139873

(56) Entgegenhaltungen:
- EP-A2- 2 439 721
- WO-A1-2014/123858
- CA-A1- 2 757 195
- DE-A1-102004 009 435
- DE-A1-102006 050 040
- US-A1- 2013 269 685

## Beschreibung

Die Erfindung betrifft einen Spender zum Austrag eines pharmazeutischen Mediums mit einem Sensor zur Erfassung eines Austrags und einer elektronischen Verarbeitungsschaltung zur Erfassung und Weiterverarbeitung eines durch den Sensor in Reaktion auf den Austragvorgang verursachten Signals.

Die Erfassung von Austragvorgängen bei solchen pharmazeutischen Spendern kann verschiedenen Zwecken dienen. Im einfachsten Falle werden die Austragvorgänge erfasst, um sie zählen zu können, so dass der Patient oder ein Arzt sich nachfolgend einen Überblick über die erfolgten Austragvorgänge verschaffen kann. Es sind jedoch auch weitergehende Verarbeitungsmöglichkeiten aus dem Stand der Technik bereits bekannt geworden, so beispielsweise die Erfassung eines Austragvorgangs zum Zwecke der Steuerung eines Blockiermechanismus, der eine Überdosierung des Mediums verhindern soll. Im Zusammenhang mit der Erfindung werden als technologischer Hintergrund die DE 10 2004 009 435 A1, DE 10 2010 042 007 A1 sowie die DE 10 2008 064 559 A1 genannt.

Obwohl die Technik zur Erfassung eines Austragvorgangs, beispielsweise in Form eines einfachen Tasters, sowie die elektronische Weiterverarbeitung dieses Austragvorgangs vergleichsweise einfache Mittel erfordert, stellt sich bei der Entwicklung derartiger Spender mitunter als Schwierigkeit dar, dass der Ort der Erfassung des Austragvorgangs nicht mit einem sinnvollen Anordnungsort für die Weiterverarbeitungsschaltung übereinstimmt. So ist beispielsweise bei einem sogenannten Metered Dose Inhaler (MDI) ein besonders sinnvoller Ort zur Erfassung des Austragvorgangs an einem unteren Ende des Gehäuses vorgesehen, wo das aus einem integrierten Container mit Auslassstutzen ausgetretene Medium in Richtung einer Austragöffnung des Spenders umgeleitet wird, während eine Verarbeitungsschaltung einschließlich einer Anzeige in Form eines LC-Displays bevorzugt auf einem Mantelkörper des Spenders angeordnet ist.

Es bereitet zum Teil einen hohen konstruktiven Aufwand, den in Hinblick auf einwandfreie Erfassung der Austragvorgänge optimiert angeordneten Sensor galvanisch mit der in Hinblick auf bequeme Handhabung angeordneten Verarbeitungsschaltung zu verbinden. Ein Verlegen entsprechender Leitungen im Spender ist meist nicht im Rahmen eines vollautomatisierten Montageprozesses möglich. Andere Techniken, wie das Aufdrucken von Leiterbahnen auf Teile des Kunststoffgehäuses eines gattungsgemäßen Spenders sind wirtschaftlich meist ebenfalls nicht zweckmäßig.

Hinzu kommt, dass Lösungen für eine solche galvanische Verbindung des Sensors mit der Verarbeitungsschaltung meist nicht zwischen Spendertypen austauschbar sind. Es bedarf somit regelmäßig für neu entwickelte Spender auch eines neuen Konzeptes in Hinblick auf die Verlegung der Signalübertragungsleitungen.

### AUFGABE UND LÖSUNG

Aufgabe der Erfindung ist es daher, einen gattungsgemäßen Spender dahingehend weiterzuentwickeln, dass dieser auf einfache Weise die Signalübertragung zwischen dem Sensor und der Verarbeitungsschaltung gestattet, wobei gewünscht ist, dass die individuellen Anpassungen an Spender unterschiedlichen Typs in Hinblick auf den Sensor, die Weiterverarbeitungsschaltung und deren Verbindung so gering als möglich ausfallen kann.

Erfindungsgemäß wird dies gemäß Anspruch 1 dadurch erreicht, dass der Sensor Teil einer Sensoreinheit ist, die einen Funksender zur Erzeugung eines Funksignals aufweist. Weiterhin ist erfindungsgemäß vorgesehen, dass die Verarbeitungsschaltung einen Funkempfänger aufweist, der zum Empfang des vom Funksender erzeugten Funksignals ausgebildet ist.

Bei einem erfindungsgemäßen Spender sind somit zwei elektronische Baueinheiten vorgesehen, die trotz ihrer Integration in einem gemeinsamen Spender miteinander nicht galvanisch gekoppelt sind. Stattdessen ist die Schnittstelle, die die elektronischen Baueinheiten, einerseits die Sensoreinheit und andererseits die Verarbeitungsschaltung, miteinander verbindet, eine Funkschnittstelle. Dies gestattet es, ohne Berücksichtigung konstruktiver Folgeproblemstellungen die jeweils ideale Anordnung zur Erfassung des Austragvorgangs und zum Verarbeiten und insbesondere Darstellen der verarbeiteten Signale zu wählen. Zu den genannten Folgeproblemen, die durch die Erfindung umgangen werden, zählen das baulich komplexe Anordnen der bisher üblicherweise erforderlichen Leitungen und das Abdichten von Wandungen, die von diesen Leitungen durchquert werden. Gerade die Möglichkeit, Teilkammer des Spenders, bspw. diejenige, die das Auswertungsmodul enthält, wasserdicht zu isolieren, ist ein wesentlicher Vorteil der Erfindung.

Die erfindungsgemäße Ausgestaltung kann bei einer Vielzahl verschiedener Typen von Spendern Verwendung finden, so beispielsweise bei den eingangs genannten "Metered Dose Inhalern", bei denen typspezifisch vorgesehen ist, dass ein durch Zusammendrücken betätigbarer Container mit Auslassstutzen in einen Aufnahmeschacht eingefügt wird und durch Niederdrücken des Containerhauptkörpers dieser gegenüber dem Auslassstutzen verlagert wird und hierdurch betätigt werden kann. Es sind jedoch auch andere Spendertypen wie beispielsweise Nasalspender und Ophthalmikspender in vorteilhafter Weise durch die erfindungsgemäßen Maßnahmen zu verbessern. Solche Spender weisen meist eine Kolbenpumpe auf, die zum Zwecke eines Flüssigkeitsaustrags manuell betätigt wird. Auch medizinische Spritzen und deren Sonderform der Autoinjektoren können erfindungsgemäß weitergebildet sein.

Die konkrete Ausgestaltung der Verarbeitungsschaltung hängt vom jeweiligen Anwendungszweck ab. Die Verarbeitungsschaltung umfasst zumindest eine Energiequelle in Form einer Batterie oder eines Akkumulators sowie den genannten Funkempfänger. Die erfindungsgemäße Ausgestaltung bietet sich insbesondere für solche Verarbeitungsschaltungen an, die zusätzlich eine Anzeigevorrichtung, beispielsweise ein LC-Display, aufweisen, da insbesondere bei solchen Verarbeitungsschaltungen die Anordnung an einer spezifischen Position des Spenders gewünscht ist. Alternativ oder zusätzlich zur Anzeigevorrichtung kann die Verarbeitungsschaltung jedoch auch einen Speicher aufweisen, um die Austragvorgänge gegebenenfalls unter Berücksichtigung des Zeitpunkts des Austragvorgangs abzuspeichern. Es sind auch Ausgestaltungen möglich, bei denen die Verarbeitungsschaltung über ein weiteres Funkmodul verfügt, welches in der Lage ist, Daten an Verarbeitungseinrichtungen jenseits des Spenders weiterzugeben. Derartige Anforderungen ergeben sich beispielsweise im Kontext der sogenannten Telemedizin.

Auf Seiten der Sensoreinheit sind zumindest der Sensor und der Funksender vorgesehen. Dabei kann der Sensor auf vielfältige Weisen ausgestaltet sein, denen gemeinsam ist, dass sie eine mechanische Größe, insbesondere eine Relativverlagerung von Bauteilen oder eine Kraft bzw. einen Druck in ein elektronisch auswertbares Signal umwandeln. Die einfachste Form eines solchen Sensors stellt einen Taster dar, der im Zuge der Betätigung des Spenders aktiviert wird. Neben den genannten Größen kann der Sensor jedoch auch auf Auswertung anderer physikalischer Größen gerichtet sein, die im Zuge eines Austragsvorgangs eine Änderung erfahren. Hierzu zählen insbesondere auch Sensoren, die den Massestrom / Volumenstrom des Mediums erfassen.

Grundsätzlich kann die Sensoreinheit ebenso wie die Verarbeitungsschaltung über eine Energiequelle in Form einer Batterie oder eines Akkumulators verfügen, der die erforderliche Energie für den Betrieb des Funksenders zur Verfügung stellt.

Ein erfindungsgemäß besonderer Vorteil ergibt sich jedoch, wenn der Sensoreinheit eine Umwandlungseinrichtung zur Umwandlung mechanischer Energie in elektrische Energie zugeordnet ist, wobei diese Umwandlungseinrichtung vorzugsweise mit dem Sensor identisch ist. Bei einer solchen Ausgestaltung wird somit unmittelbar die vom Benutzer in das System im Zuge des Austragvorgangs eingebrachte Energie genutzt, um diese zumindest teilweise in elektrische Energie zu wandeln, welche anschließend den Funksender versorgen kann. Eine solche Umwandlungseinrichtung stellt vorzugsweise selbst den Sensor dar. Wenn die Umwandlungseinrichtung also elektrische Energie zur Verfügung stellt, so geschieht dies in Reaktion auf einen erfolgenden Austragvorgang. Die Sensoreinheit kann demnach so ausgebildet sein, dass sie beim Vorliegen dieser elektrischen Energie unmittelbar das Funksignal abgibt.

Als Umwandlungseinrichtung kommt insbesondere eine piezoelektrische Umwandlungseinrichtung infrage, insbesondere in Form eines sogenannten Piezostapels, der als Piezogenerator arbeitet. Der Verzicht auf eine Batterie oder einen Akkumulator auf Seiten der Sensoreinheit ist insbesondere aufgrund der leichteren Entsorgbarkeit des Spenders nach Gebrauch vorteilhaft.

Um trotz der zwingend erforderlichen Stromquelle auf Seiten der Verarbeitungsschaltung die einfache Entsorgbarkeit zu gewährleisten, kann die Verarbeitungsschaltung als durch den Endverbraucher lösbare Einheit am Spender vorgesehen sein, welche vor Entsorgung der übrigen Teile des Spenders von diesem gelöst wird und einer separaten Entsorgung oder einer Wiederverwertung zugeführt wird.

Die Verarbeitungsschaltung und die Sensoreinheit sind bestimmungsgemäß fest mit Teilkomponenten des Spenders verbunden und vorzugsweise auch zueinander ortsfest angeordnet. Um mit einer besonders geringen Sendeleistung auf Seiten der Sensoreinheit auszukommen, und insbesondere, um einen nicht amplituden- oder frequenzmodulierten sehr einfachen Funkimpuls zur Signalübertragung nutzen zu können, ist es von Vorteil, wenn der Funksender und der Funkempfänger nicht mehr als 10 cm voneinander entfernt am Spender angeordnet sind. Der bevorzugte Frequenzbereich, in dem der Funksender und dem Funkempfänger arbeiten, liegt zwischen 100 kHz und 3GHz.

Besonders vorteilhaft ist, insbesondere bei MDIs, die folgende Anordnung. Der Spender weist einen Aufnahmeschacht auf, in dem der Container angeordnet ist. Dieser Aufnahmeschacht ist durch eine Wandung umgeben. Der genannte Sensor befindet sich innerhalb des Aufnahmeschachtes, worunter verstanden wird, dass bezogen auf die Erstreckungsrichtung des Schachtes innerhalb von dessen Flucht angeordnet ist. Die Verarbeitungsschaltung ist bezogen auf die Projektion des Schachtes außerhalb desselben angeordnet und vorzugsweise an der Außenseite der Wandung des Schachtes befestigt. Dies ist eine bei MDIs übliche Gestaltung, bei der die Anordnung der Leitungen in der Vergangenheit Schwierigkeiten bereitet hat. Gerade bei einer solchen Anordnung ist die vorgeschlagene Funkverbindung daher vorteilhaft.

Insbesondere bei der genannten Ausgestaltung der Sensoreinheit mit einer Umwandlungseinrichtung, die den Funksender mit elektrischer Energie versorgt, ist es von Vorteil, die Signalübertragung mittels eines solch einfachen Funkimpulses zu bewerkstelligen. Hierfür kann beispielsweise ein einfacher Schwingkreis, rein beispielhaft mit einer Resonanzfrequenz von beispielsweise 1 GHz, genutzt werden. Wenn die Übertragung von mehr als nur einem Impuls gewünscht ist, ist es von Vorteil, wenn auf ein standardisiertes Funkübertragungsverfahren zurückgegriffen wird, insbesondere nach dem Bluetooth-Standard, nach dem Zigbee-Standard, nach dem Ant Plus-Standard, nach dem Wibree-Standard oder nach dem IEEE 802.15.4-Standard. Diese Standards sind jeweils darauf ausgerichtet, dass Sender und Empfänger mit einem geringen Maß an elektrischer Energie auskommen. Die Nutzung derartiger Standards gestattet eine bidirektionale Kommunikation zwischen der Sensoreinheit und der Verarbeitungsschaltung, was das Anwendungsspektrum erfindungsgemäßer Spender vergrößern kann.

### KURZBESCHREIBUNG DER ZEICHNUNGEN

Weitere Aspekte und Vorteile ergeben sich aus den Ansprüchen und der nachfolgenden Beschreibung zweier Ausführungsbeispiele der Erfindung, die anhand der Figuren erläutert werden. Dabei zeigen:
- Fig. 1: eine erste Ausführungsform eines erfindungsgemäßen Spenders und
- Fig. 2: eine zweite Ausführungsform eines erfindungsgemäßen Spenders.

### DETAILLIERTE BESCHREIBUNG DER AUSFÜHRUNGSBEISPIELE

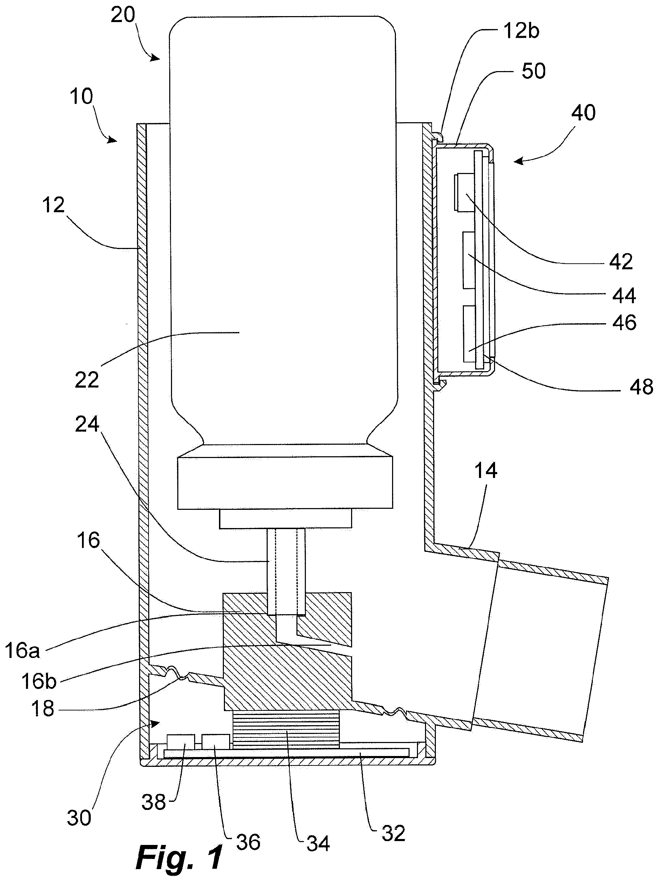

Fig. 1 zeigt eine erste Ausführungsform eines erfindungsgemäßen Spenders. Dieser ist in Art eines MDI (Metered Dose Inhaler) ausgestaltet. Er verfügt über ein Kunststoffgehäuse 10, welches einen in etwa zylindrischen Aufnahmebereich 12 sowie ein hiergegen angewinkeltes Mundstück 14 aufweist. Der Aufnahmebereich 12 ist zur Aufnahme eines Container 20 ausgebildet. Dieser Container 20 wiederum verfügt über einen Flüssigkeitsspeicher 22 und einen Auslassstutzen 24, die gegeneinander verlagerbar sind. Dabei ist der Container derart ausgebildet, dass durch Niederdrücken des Flüssigkeitsspeichers 22 eine Relativverlagerung gegenüber dem Auslassstutzen 24 stattfindet, durch die eine definierte Menge des zuvor im Flüssigkeitsspeicher 22 gelagerten Mediums durch den Auslassstutzen 24 hinweg ausgetragen wird. Der Auslassstutzen 24 ist in einer zum Gehäuse 10 gehörigen Aufnahme 16a aufgenommen, an die sich ein Austragkanal 16b anschließt. Die Aufnahme 16a und der Austragkanal 16b sind in einem begrenzt verlagerbaren Gehäuseabschnitt 16 vorgesehen, der mittels eines dünnwandigen Anbindungsbereichs 18 mit umgebenden Teilen des Gehäuses 10 verbunden ist. Der Gehäuseabschnitt 16 ist daher gegenüber dem zylindrischen Bereich 12 und dem Mundstück 14 des Spenders begrenzt beweglich.

Zur Erfassung von Austragvorgängen verfügt der Spender über eine Sensoreinheit 30, welche unterhalb des beweglichen Gehäuseabschnitts 16 an das Gehäuse 10 angefügt ist. Diese Sensoreinheit 30 umfasst eine Platine 32, auf die ein Piezostapel 34 aufgesetzt ist, der unmittelbar unterhalb des Gehäuseabschnitts 16 angeordnet ist. Mit dem Piezostapel 34 ist ein Energiezwischenspeicher 36, beispielsweise in Art eines Kondensators, verbunden. Dieser wiederum ist mit einem einfachen Funksender 38 in Art eines 1-GHz-Schwingkreises gekoppelt.

An der äußeren Mantelfläche des zylindrischen Aufnahmebereichs 12 des Gehäuses 10 ist eine Verarbeitungsschaltung 40 vorgesehen, welche über eine Energiequelle 42 in Form einer Knopfzelle, über ein Verarbeitungsschaltkreis 44 und über einen Funkempfänger 46 verfügt. Zusätzlich ist ein LC-Display 48 vorgesehen, welches mit dem Schaltkreis 44 verbunden ist. Die Verarbeitungsschaltung 40 ist in ein Gehäuse 50 eingesetzt, welches an der Außenseite des zylindrischen Aufnahmebereichs 12 in einer Halterung 12b eingeschoben ist.

Bei einer Betätigung des Spenders durch Hinabdrücken des Flüssigkeitsspeichers 22 kommt es zu einer Verlagerung des Auslassstutzens 24 gegenüber dem Flüssigkeitsspeicher 22 sowie zu einer Verlagerung des Gehäuseabschnitts 16 bei gleichzeitigem Zusammendrücken des Piezostapels 34. Die Komprimierung des Piezostapels 34 führt dazu, dass elektrische Energie erzeugt wird, die im Kondensator 36 gespeichert wird. Der Container 20 und die Sensoreinheit 30 sind derart aufeinander abgestimmt, dass die durch den Piezostapel 34 erzeugte Energie im Kondensator 36 ein zum Betrieb des Funksenders 38 ausreichendes Energieniveau erreicht, kurz bevor die Verlagerung des Flüssigkeitsspeichers 22 gegenüber dem Auslassstutzen 24 ausreichend groß ist, um den Austragvorgang zu bewirken. Kurz vor dem Austragvorgang kommt es somit zur Abgabe eines Funkimpulses durch den Funksender 38.

Dieser Funkimpuls wird vom Funkempfänger 46 empfangen und an den Schaltkreis 44 weitergegeben. Dieser addiert auf die darin gespeicherte Anzahl vorheriger Austragvorgänge den nun registrierten und zeigt das Ergebnis für einen kurzen Zeitraum von einigen Sekunden oder Minuten auf dem LC-Display 48 an.

Durch die Gestaltung des Systems mit einem Funksender 38 und einem Funkempfänger 46 wird erreicht, dass eine optimale Anordnung sowohl des Sensors 34 als auch des Displays 48 möglich ist, ohne dass die hierbei üblicherweise herrschende Problematik der Anordnung von Leitungen berücksichtigt werden muss.

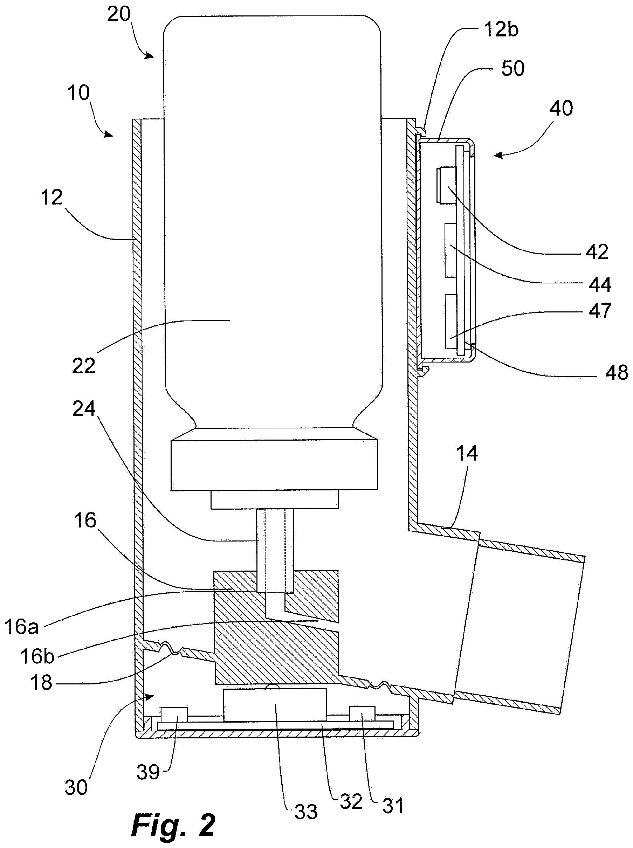

Die Ausführungsform der Fig. 2 ist hinsichtlich der meisten Aspekte identisch mit jener der Fig. 1. Der wesentliche Unterschied liegt darin, dass bei dieser zweiten Ausführungsform auch die Sensoreinheit 30 über eine Energiequelle 31 in Form einer Batterie verfügt und dass statt des Piezostapels ein einfacher Taster 33 vorgesehen ist. Weiterhin sind der Funksender 39 und der Funkempfänger 47 bei diesem zweiten Ausführungsbeispiel als bidirektional kommunizierende Bluetooth-Funkeinheiten ausgestaltet.

Die elektrische Energie zum Betreiben der sensoreinheitsseitigen Funkeinrichtung 39 muss somit nicht durch den Austragvorgang und die damit ins System eingebrachte mechanische Energie in umgewandelter Form zur Verfügung gestellt werden, sondern entstammt direkt der Batterie 31.

## Patentansprüche

1. Spender zum Austrag eines pharmazeutischen Mediums mit
- einem Sensor (33; 34) zur Erfassung eines Austragvorgangs und
- einer elektronischer Verarbeitungsschaltung (40) zur Erfassung und Weiterverarbeitung eines durch den Sensor (33;34) verursachten Signals,
wobei
- der Sensor (33; 34) Teil einer Sensoreinheit (30) ist, die eine Funksender (38; 39) zur Erzeugung eines Funksignals aufweist, und
- die Verarbeitungsschaltung (40) einen Funkempfänger (46; 47) aufweist, der zum Empfang des vom Funksender (38; 39) erzeugten Funksignals ausgebildet ist,
**dadurch gekennzeichnet, dass**
der Sensoreinheit (30) eine Umwandlungseinrichtung (34) zur Umwandung mechanischer Energie in elektrische Energie derart zugeordnet ist, dass die von einem Benutzer im Zuge eines Austragvorgangs eingebrachte mechanische Energie genutzt wird, um diese zumindest teilweise in elektrische Energie zu wandeln, welche anschließend den Funksender versorgen kann.

2. Spender nach Anspruch 1,
**dadurch gekennzeichnet, dass**
die Umwandungseinrichtung (34) mit dem Sensor (34) identisch ist.

3. Spender nach Anspruch 1 oder 2,
**dadurch gekennzeichnet, dass**
der Funksender (38; 39) und der Funkempfänger (46; 47) nicht mehr als 10 cm voneinander entfernt angeordnet sind.

4. Spender nach einem der vorstehenden Ansprüche,
**dadurch gekennzeichnet, dass**
der Spender einen Aufnahmeschacht (12) aufweist, in den ein Container (20) eingesetzt ist, wobei zumindest der Sensor innerhalb des Aufnahmeschachtes (12) angeordnet ist und wobei zumindest die Verarbeitungsschaltung außerhalb des Aufnahmeschachtes (12) angeordnet ist.

5. Spender nach einem der vorstehenden Ansprüche
**dadurch gekennzeichnet, dass**
der Funksender (39) zur Erzeugung eines Funksignals nach dem Bluetooth-Standard, dem Wibree-Standard, dem ANT+-Standard oder dem Zigbee-Standard ausgebildet ist.

6. Spender nach einem der vorstehenden Ansprüche,
**dadurch gekennzeichnet, dass**
die elektronische Schaltung (40) zum Zählen von Austragvorgängen ausgebildet ist.

7. Spender nach einem der vorstehenden Ansprüche,
**dadurch gekennzeichnet, dass**
der Sensor (34; 33) zur Erfassung
- der Bewegung einer Betätigungshandhabe oder eines mit der Betätigungshandhabe mechanische gekoppelten Bauelements gegenüber einem Gehäuse ausgebildet ist oder
- der Bewegung eines in das Gehäuse des Spenders eingesetzten Containers (20) ausgebildet ist.

## Claims

1. Dispenser for dispensing a pharmaceutical medium, comprising
- a sensor (33; 34) for sensing a dispensing operation and
- an electronic processing circuit (40) for sensing and further processing a signal caused by the sensor (33; 34),
wherein
- the sensor (33; 34) is part of a sensor unit (30), which has a radio transmitter (38; 39) for producing a radio signal, and
- the processing circuit (40) has a radio receiver (46; 47), which is designed for receiving the radio signal produced by the radio transmitter (38; 39),
**characterized in that**
the sensor unit (30) is assigned a conversion device (34) for the conversion of mechanical energy into electrical energy such that the mechanical energy that is introduced by a user in the course of a dispensing operation is used to convert it at least partially into electrical energy, which can subsequently supply the radio transmitter.

2. Dispenser according to Claim 1,
**characterized in that**
the conversion device (34) is identical to the sensor (34).

3. Dispenser according to Claim 1 or 2,
**characterized in that**
the radio transmitter (38; 39) and the radio receiver (46; 47) are arranged no more than 10 cm apart from one another.

4. Dispenser according to one of the preceding claims,
**characterized in that**
the dispenser has a receiving shaft (12), in which a container (20) is inserted, at least the sensor being arranged within the receiving shaft (12) and at least the processing circuit being arranged outside the receiving shaft (12).

5. Dispenser according to one of the preceding claims,
**characterized in that**
the radio transmitter (39) is designed for producing a radio signal on the basis of the Bluetooth standard, the Wibree standard, the ANT+ standard or the Zigbee standard.

6. Dispenser according to one of the preceding claims,
**characterized in that**
the electronic circuit (40) is designed for counting dispensing operations.

7. Dispenser according to one of the preceding claims,
**characterized in that**
the sensor (34; 33) is designed for sensing
- the movement of an actuating handle or a structural element mechanically coupled to the actuating handle with respect to a housing or
- the movement of a container (20) inserted in the housing of the dispenser.

## Revendications

1. Distributeur pour distribution d'un support pharmaceutique avec
- un capteur (33 ; 34) pour enregistrer la distribution et
- un circuit de traitement électronique (40) pour enregistrer et traiter ultérieurement un signal provenant du capteur (33 ; 34),
dans lequel
- le capteur (33 ; 34) fait partie d'une unité de détection (30), comportant un émetteur radio (38 ; 39) produisant un signal radio, et
- le circuit de traitement (40) comporte un récepteur radio (46 ; 47), conçu pour recevoir le signal radio produit par l'émetteur radio (38 ; 39),
**caractérisé en ce que**
l'unité de détection (30) est ordonnée à un dispositif de conversion (34) pour la conversion de l'énergie mécanique en énergie électrique de telle manière que l'énergie mécanique fournie par un utilisateur au cours d'une distribution est utilisée pour être convertie au moins en partie en énergie électrique, laquelle peut alimenter ensuite l'émetteur radio.

2. Distributeur selon la revendication 1, **caractérisé en ce que** l'unité de conversion (34) est identique au capteur (34).

3. Distributeur selon la revendication 1 ou 2, **caractérisé en ce que** l'émetteur radio (38 ; 39) et le récepteur radio (46 ; 47) sont agencés en n'étant pas éloignés de plus de 10 cm l'un de l'autre.

4. Distributeur selon l'une des revendications précédentes, **caractérisé en ce que** le distributeur comporte un puits de réception (12), où un conteneur (20) est inséré, dans lequel au moins le capteur est agencé à l'intérieur du puits de réception (12) et dans lequel au moins le circuit de traitement est agencé à l'extérieur du puits de réception (12).

5. Distributeur selon l'une des revendications précédentes, **caractérisé en ce que** l'émetteur radio (39) est conçu pour produire un signal radio selon le standard Bluetooth, le standard Wibree, le standard ANT+ ou le standard Zigbee.

6. Distributeur selon l'une des revendications précédentes, **caractérisé en ce que** le circuit électronique (40) est conçu pour compter les distributions.

7. Distributeur selon l'une des revendications précédentes, **caractérisé en ce que** le capteur (34 ; 33) est conçu pour enregistrer
- le mouvement d'une poignée ou d'un composant raccordé mécaniquement à la poignée par rapport à un boîtier ou
- le mouvement d'un conteneur (20) agencé dans le boîtier du distributeur.
